Europäisches Patentamt

European Patent Office

Office européen des brevets

(11) Veröffentlichungsnummer: **0 298 355 B1**

(12) **EUROPÄISCHE PATENTSCHRIFT**

(45) Veröffentlichungstag der Patentschrift:
14.11.90

(51) Int. Cl.⁵: **B01J 2/30**, B01J 2/14, A61K 9/50

(21) Anmeldenummer: 88110355.0

(22) Anmeldetag: 29.06.88

(54) **Verfahren zur Umhüllung von Granulaten.**

(30) Priorität: 01.07.87 DE 3721721

(43) Veröffentlichungstag der Anmeldung:
11.01.89 Patentblatt 89/2

(45) Bekanntmachung des Hinweises auf die Patenterteilung:
14.11.90 Patentblatt 90/46

(84) Benannte Vertragsstaaten:
AT BE CH DE ES FR GB GR IT LI LU NL SE

(56) Entgegenhaltungen:
DE-A- 2 122 355
DE-A- 2 436 052
FR-A- 1 077 950
FR-A- 1 583 182
US-A- 4 132 753

(73) Patentinhaber: HOECHST AKTIENGESELLSCHAFT,
Postfach 80 03 20, D-6230 Frankfurt am Main 80(DE)

(72) Erfinder: Ahrens, Gerhard Dr., Am Flachsland 54,
D-6233 Kelkheim Taunus(DE)

ACTORUM AG

## Beschreibung

Die Erfindung betrifft ein Verfahren zur Umhüllung von Granulaten mit ausschließlich pulverförmigen Substanzen sowie die nach diesem Verfahren erhaltenen umhüllten Granulate. Die Granulate finden insbesondere Verwendung auf dem Arzneimittelsektor.

Die Umhüllung sphärischer Granulate, die meistens zum Zwecke der Regulierung der Wirkstofffreigabe oder zur Kaschierung eines unvorteilhaften Geschmackes oder zur Erzielung von Resistenz gegen Verdauungssäfte bzw. Stabilisierung gegen Umgebungseinflüsse vorgenommen wird, erfolgt überwiegend durch Aufgießen oder Aufsprühen von Lösungen, Suspensionen, Emulsionen oder Schmelzen der Hüllsubstanzen.

Bei der von einem Starterkorn ausgehenden Aufbaugranulation, die ebenfalls als Umhüllung von Granulaten angesehen werden kann, erfolgt die Zugabe des zur Granulation vorgesehenen Materials zwar in Pulverform doch ist auch hier ein flüssiges Medium als Bindemittel oder Träger für ein Bindemittel unerläßlich.

Das Aufbringen flüssiger Medien bei der Umhüllung von Granulaten erfolgt durch Aufgießen oder Aufsprühen, vorteilhafterweise jedoch durch Aufsprühen. Zum Versprühen sind zumindestens im Falle vom Schmelzen aufwendige Aggregate notwendig. Werden Lösungen, Suspensionen oder Emulsionen aufgebracht, so muß flüssiger Hilfsstoff zeitaufwendig wieder entfernt und in vielen Fällen wegen der umweltgefährdenden Eigenschaften der verwendeten Stoffe unter großem Kostenaufwand entsorgt werden. So sind insbesondere bei gut wasserlöslichen Wirkstoffen größere Mengen organischer Lösungsmittel beim Auftragen der Abschlußmembran erforderlich. Der Auftrag der Hüllsubstanzen auf Granulate ausschließlich in Pulverform ohne Verwendung flüssiger Hilfsstoffe, der die obengenannten Nachteile vermeiden ließe, ist bisher nicht beschrieben. Zwar ist die elektrostatische Pulverlackierung seit langem bekannt und weit verbreitet, doch die Umsetzung in ein praxisgerechtes Verfahren zur Umhüllung von Granulaten steht noch aus.

Die Verwendung von als Rotorgranulator (K.H. Bauer, Pharm. Ind. _41_, 973, (1979)) oder Zentrifugalgranulator (Y. Funakoshi, Powder Technol. _27_, 13, (1980)) bezeichneten Geräten für die Umhüllung von Granulaten ist wohlbekannt. Die genannten Geräte bestehen im wesentlichen aus einem vertikal angeordneten runden Behältnis, in dem sich eine horizontal angebrachte Scheibe befindet, mit der das Granulat rotiert, während der Behälter unbewegt ist. Der schmale Spalt zwischen rotierender Scheibe und Behälterwand wird von Luft durchströmt, die zum einen ein Durchfallen des Materials verhindert, zum anderen auch aufgesprühte Lösemittel abführt. Spheronizer wie z.B. ®Marumerizer (Reynolds A.D., Manufacturing Chemist. _41_, 40, (1970)), aus denen die Rotorgranulatoren hervorgingen, haben keine Einrichtungen für die Zu- und Abfuhr großer Mengen Trocknungsluft. Sie wurden für die Ausrundung von Partikeln plastischer Massen entwickelt.

Die Verwendung wachsartiger Substanzen zur Umhüllung von Granulaten (vgl. z.B. Japanische Patentanmeldung Nr. 53062821, Deutsche Offenlegungsschrift 26 51 176 (= GB-Patent No. 1560841) US-Patent Nr. 3 119 742 und Britische Patentschrift Nr. 1 044 572) ist ebenfalls seit langem bekannt, wobei jedoch auf flüssige Zubereitungen der Hüllsubstanzen nicht verzichtet werden konnte.

Es wurde nun überraschenderweise gefunden, daß Granulate unter ausschließlicher Verwendung pulvriger Substanzen umhüllt werden können.

Voraussetzung für jeden Auftrag pulvriger Substanz auf Formlinge ist es, für eine ausreichende Haftung auf der zu beschichteten Oberfläche zu sorgen. Bei der Aufbaugranulation wird hierzu, wie erwähnt, eine flüssige Bindemittelzubereitung verwendet. Das Problem wird durch die vorliegende Erfindung auf sehr elegante Weise gelöst, indem in das zu beschichtende Granulat eine wachsartige Substanz inkorporiert wird und das Granulat auf der rotierenden Scheibe unter Wärmezufuhr bewegt wird. Durch die Erweichung des wachsartigen Hilfsstoffes im zu beschichtenden Granulat wird dessen Oberfläche klebrig und ist bereit zur Aufnahme der pulvrigen Hüllsubstanzen ohne Zufuhr von Flüssigkeit und ohne daß es zur Verklebung der Partikeln untereinander kommt.

Die Erfindung betrifft daher ein Verfahren zur Umhüllung von ausgerundeten oder nicht ausgerundeten, auf herkömmliche Weise hergestellten, einen Gehalt an wachsartigen Substanzen aufweisenden Granulaten in einer Apparatur mit einer in einem vertikal angeordneten Behälter rasch rotierenden, horizontal angeordneten Scheibe mit einer oder mehreren wachsartigen Substanzen oder einem Gemisch aus einer oder mehreren wachsartigen Substanzen, dadurch gekennzeichnet, daß man auf die Granulate, die bis zur Erweichung der wachsartigen Substanz erhitzt worden sind, die wachsartigen Substanzen in Pulverform aufstreut. Die wachsartigen Substanzen können gegebenenfalls im Gemisch mit einem oder mehreren Wirkstoffen und gegebenenfalls einem oder mehreren nicht wachsartigen Hilfsstoffen unter Rotieren der Scheibe aufgestreut werden.

Das Verfahren wird vorzugsweise in einem Spheronizer (z.B. Marumerizer), einem Rotor- oder Zentrifugal-Granulator durchgeführt. Zur Erwärmung setzt man z.B. einen Heißluftstrom oder einen Infrarotstrahler ein.

In den vorstehenden und folgenden Ausführungen werden unter wachsartigen Substanzen natürliche, halbsynthetische und synthetische Wachse verstanden. Sie haben hydrophobe oder hydrophile Eigenschaften. Durch den Gehalt an wachsartiger Substanz oder eines Gemisches wachsartiger Substanzen im Granulat oder in der Umhüllung wird die Wirkstofffreigabe beeinflußt; sie kann durch geeignete Substanzen verzögert werden. Als wachsartige Hüllsubstanzen kommen insbesondere Fettalkohole und deren Derivate, Fettsäuren und deren Derivate, Kohlenwasserstoffe, Wachse und Polyethylenglykole und deren Derivate mit einem Schmelzpunkt oberhalb 20°C in Betracht.

Das auf herkömmliche Weise hergestellte Granulat ist z.B. ein Schmelzgranulat, ein Trockengranulat, ein Feuchtgranulat oder es handelt sich um Stränglinge, die durch Extrusion entstehen.

Das zu umhüllende Granulat enthält außer dem wachsartigen Hilfsstoff auch den oder die Wirkstoffe. Auch das pulverförmige wachsartige Umhüllungsmaterial kann einen oder mehrere Wirkstoffe enthalten. Umhüllung und Granulat können gleiche oder verschiedene Wirkstoffe enthalten. Das erfindungsgemäße Verfahren eignet sich insbesondere zur Umhüllung von Granulaten leicht wasserlöslicher Wirkstoffe.

Sowohl Granulat als auch das Umhüllungsmaterial können weitere allgemein übliche nicht wachsartige Hilfsstoffe enthalten, die wasserlöslich oder wasserunlöslich sind. Sie können auch in Wasser quellbare Stoffe sein.

Bei der Durchführung des Verfahrens ist es zweckmäßig, daß die zu umhüllenden Granulatpartikeln vor dem Umhüllungsschritt durch längeres Rotieren und Erwärmen unter plastischer Verformung in eine sphärische Form gebracht werden. Dies ist insbesondere dann zweckmäßig, wenn es auf eine gleichmäßige Schichtdicke der Hülle ankommt.

Eine weitere Voraussetzung für das Gelingen des Pulverauftrags ist die sehr rasche Verteilung der Hüllsubstanzen über die gesamte zu beschichtende Oberfläche, was bei dem vorliegenden Verfahren durch die Verwendung der rotierenden Scheibe gelöst wird. So kann es nicht zur Selbstagglomeration des eingestreuten Pulvers kommen, was im langsam rotierenden Kessel der Fall wäre.

Die auf das Granulat locker aufgetragene Pulverschicht muß, besonders wenn sie Membranfunktion übernehmen soll, in eine dichte zusammenhängende Schicht überführt werden. Des weiteren muß die nachfolgende Pulverschicht Haftung finden.

Beide Probleme werden durch die Inkorporierung wachsartiger Substanz in die Mischung der Hüllsubstanzen gelöst. Wärmezufuhr führt zur Erweichung des Wachsanteils, der unter Rotieren zu einer dichten Hülle zusammensintert und auch der nächsten Schicht Haftung bietet.

Wenn die nach dem erfindungsgemäßen Verfahren aufzutragende Hülle zur Regulierung der Wirkstofffreigabe aus dem Granulat, oder zur Kaschierung eines unvorteilhaften Geschmacks oder zur Erzielung von Resistenz gegen Verdauungssäfte bzw. zur Stabilisierung gegen Umgebungseinflüsse dient, ist es oft wünschenswert, daß das nicht umhüllte Granulatkorn eine nicht verzögerte Wirkstofffreigabe aufweist. Die für die Durchführung des Prozesses notwendige wachsartige Substanz im Innern des Granulatkorns darf daher in solchen Fällen nicht freigabeverzögernd wirken. Polyethylenglykole, die in dieser Hinsicht in Frage kämen, eignen sich allein dafür nicht, denn damit hergestellte Granulate lassen sich auf der rotierenden Scheibe unter Wärmeeinwirkung nicht ohne Verkleben durch plastische Verformung ausrunden. Zudem haftet das Gemisch der Hüllsubstanzen, das zur Erzielung der obengenannten Zwecke hydrophobe wachsartige Substanzen enthält, nicht auf der Oberfläche solcher Granulate. Es wurde nun gefunden, daß man Granulate erhält, die zum einen sehr gut für den Prozeß geeignet sind, zum anderen aber keine verzögerte Wirkstofffreigabe aufweisen, wenn man sowohl Polyethylenglykole als hydrophile wachsartige Substanzen als auch hydrophobe wachsartige Substanzen, wie sie im Gemisch der Hüllsubstanzen enthalten sind, in das Granulat inkorporiert.

Der Verfahrensablauf im einzelnen sieht z.B. so aus, daß das zur Umhüllung vorgesehene Granulat, das auf herkömmliche Weise hergestellt wird, in einem Gerät nach Art eines Spheronizers rotiert wird und dabei durch einen Heißluftstrom oder Bestrahlung mit einer Infrarotlampe bis zur beginnenden Erweichung erhitzt wird. Dieser Zeitpunkt ist im allgemeinen an einer Formänderung des rotierenden Gutbettes erkennbar. Es nimmt dabei eine schlauchförmige Form an. Das weitere Erhitzen wird sehr vorsichtig vorgenommen, um ein Zusammenkleben der Partikeln zu verhindern. Bei Bedarf wird das Rotieren unter wohldosierter Wärmezufuhr fortgesetzt bis der gewünschte Ausrundungszustand erreicht ist.

Auf das nicht ausgerundete oder wie oben ausgerundete Granulat, das bis zur beginnenden Erweichung erhitzt wurde, wird ein Pulver aus reiner wachsartiger Substanz, oder aus reinen wachsartigen Substanzen, bzw. ein Pulvergemisch aus Wirkstoffen und wachsartiger Substanz oder ein Pulvergemisch aus wachsartiger Substanz und nicht wachsartigen Hilfsstoffen und gegebenenfalls Wirkstoffen aufgetragen. Durch alternierendes Einstreuen und Erhitzen bzw. gleichzeitiges Einstreuen und Erhitzen bei getrennter Positionierung von Wärme- und Pulverzufuhr können die jeweiligen Schichten bis zur gewünschten Dicke aufgebaut werden.

Beispiel 1:

Ein Schmelzgranulat (Fraktion 0,75–1,25 mm) aus
100 g Furosemid
100 g Hartparaffin (Spezialwachs 4900) und
100 g Hydroxypropyl-methylcellulose (®Methocel K15M) wurde in einem Spheronizer mit ca. 22 cm Scheibendurchmesser bei etwa 250 Upm rotiert und mit einer handelsüblichen Heißluftpistole erhitzt bis es zur beginnenden Erweichung des Materials kam, erkenntlich am veränderten Laufverhalten des Gutes. Anschließend wurde die Umdrehungsgeschwindigkeit auf ca. 800 Upm erhöht und eine Teilmenge einer Pulvermischung aus

200 g Furosemid
200 g Hartparaffin (Spezialwachs (4900)
200 g Hydroxypropyl-methylcellulose (Methocel K15M) und
30 g Natriumdihydrogenphosphat

so lange aufgestreut, bis das leichte Kleben der Granulatkörner, erkenntlich am besonderen Laufverhalten des Gutbettes, aufgehört hatte. Es wurde anschließend erneut bis zum beginnenden Kleben der Partikeln erhitzt und wiederum Pulvermischung eingestreut. Der Prozeß wurde bis zum Auftrag der gesamten Pulvermenge fortgesetzt.

Die Siebfraktion von 1,0–1,25 mm wurde in einer paddle-Apparatur nach USP XXI auf Wirkstofffreigabe untersucht:
Einwaage: 184,5 mg sphärisches Granulat
Rührgeschwindigkeit: 50 Upm
Auflösungsmedium: Phospatpuffer pH 5,8
Es wurden an Furosemid kumulativ freigesetzt:
1. Stunde 7,0 %
2. Stunde 20,9 %
3. Stunde 36,0 %
4. Stunde 48,6 %
5. Stunde 61,6 %
6. Stunde 71,9 %

**Beispiel 2:**

Ein Schmelzgranulat (Fraktion 0,75 - 1,25 mm) aus
225 g Metamizol
39,6 g Hartparaffin (Spezialwachs 4900) und
35,4 g Polyethylenglykol 6000
wurde unter gleichen Bedingungen wie in Beispiel 1 unter Rotieren erhitzt und eine erste Pulvermischung aus
45 g Metamizol
7,05 g Hartparaffin (Spezialwachs 4900) und
7,95 g Polyethylenglykol 6000
aufgetragen.
Anschließend wurde, wiederum unter gleichen Bedingungen, eine zweite Pulvermischung aus
75 g Hartparaffin (Spezialwachs 4900)
24 g mikronisierter Lactose und
1 g Eisenoxidrot aufgetragen.
Die Siebfraktion 1,0 - 1,25 mm wurde in einer basket -Apparatur nach USP XXI auf Wirkstofffreigabe untersucht:
Einwaage: 127,77 mg sphärisches Granulat
Rührgeschwindigkeit: 100 Upm
Auflösungsmedium: künstl. Magensaft pH 1,2
Es wurde an Metamizol kumulativ freigesetzt:

1. Stunde 8,0 %
2. Stunde 17,2 %
3. Stunde 28,8 %
4. Stunde 40,9 %
5. Stunde 52,1 %
6. Stunde 61,3 %.

**Beispiel 3:**

Es wurde bis einschließlich des Auftrages der ersten Pulvermischung wie in Beispiel 2 verfahren und anschließend unter gleichen Bedingungen wie in Beispiel 2 eine zweite Pulvermischung aus

50 g Hartparaffin (Spezialwachs 4900)
16 g mikronisierter Lactose und
0,66 g Eisenoxidrot aufgetragen.
Die Wirkstofffreigabe wurde unter gleichen Bedingungen wie in Beispiel 2 bestimmt:
Einwaage: 118,52 g sphärisches Granulat

1. Stunde 17,7 %
2. Stunde 34,6 %
3. Stunde 49,3 %
4. Stunde 64,0 %
5. Stunde 74,1 ‰
6. Stunde 79,0 %.

**Patentansprüche**

1. Verfahren zur Umhüllung von ausgerundeten oder nicht ausgerundeten, auf herkömmliche Weise hergestellten, einen Gehalt an wachsartigen Substanzen aufweisenden Granulaten in einer Apparatur mit einer in einem vertikal angeordneten Behälter rasch rotierenden, horizontal angeordneten Scheibe mit einer oder mehreren wachsartigen Substanzen oder einem Gemisch aus einer oder mehreren wachsartigen Substanzen, dadurch gekennzeichnet, daß man auf die Granulate, die bis zur Erweichung der wachsartigen Substanz erhitzt worden sind, die wachsartigen Substanzen in Pulverform aufstreut.

2. Verfahren nach Anspruch 1, dadurch gekennzeichnet, daß man die wachsartigen Substanzen im Gemisch mit einem oder mehreren Wirkstoffen und gegebenenfalls einem oder mehreren nicht wachsartigen Hilfsstoffen in Pulverform aufstreut.

3. Verfahren nach Anspruch 1, dadurch gekennzeichnet, daß man die Granulate vor der Umhüllung durch längeres Rotieren und Erwärmen unter plastischer Verformung in eine sphärische Form bringt.

4. Verfahren nach Anspruch 1, dadurch gekennzeichnet, daß die zu umhüllenden Granulate eine hydrophobe wachsartige Substanz oder eine Mischung aus hydrophilen und hydrophoben wachsartigen Substanzen enthalten.

5. Verfahren nach Anspruch 1, dadurch gekennzeichnet, daß man als wachsartige Substanz Fettalkohole oder deren Derivate, Fettsäuren und deren Derivate, Kohlenwasserstoffe, Wachse oder Polyethylenglykole und deren Derivate mit einem Schmelzpunkt oberhalb 20°C einsetzt.

6. Granulate mit wachsartiger Umhüllung erhältlich nach Verfahren gemäß Anspruch 1.

**Claims**

1. A process for coating rounded or non-rounded granules, prepared in the conventional manner and containing wax-like substances, in an apparatus having a rapidly rotating horizontal disk located in a vertical container, in the presence of one or more wax-like substances or a mixture of one or more wax-like substances, which comprises scattering the wax-like substances in powder form onto the granules which have been heated until the wax-like substance has softened.

2. The process as claimed in claim 1, wherein the wax-like substances are scattered in powder form in admixture with one or more active compounds and, if desired, one or more non-wax-like auxiliaries.

3. The process as claimed in claim 1, wherein the granules are given a spherical shape, prior to the coating, by prolonged rotation and heating with plastic deformation.

4. The process as claimed in claim 1, wherein the granules to be coated contain a hydrophobic wax-like substance or a mixture of hydrophilic and hydrophobic wax-like substances.

5. The process as claimed in claim 1, wherein fatty alcohols or their derivatives, fatty acids and their derivatives, hydrocarbons, waxes or polyethylene glycols and their derivatives having a melting point in excess of 20°C are used as the wax-like substance.

## Revendications

1. Procédé d'enrobage de granulés arrondis ou non, fabriqués de manière classique et comportant une certaine quantité de substances cireuses, dans un appareillage pourvu d'un disque placé horizontalement tournant rapidement dans un récipient disposé verticalement et contenant une ou plusieurs substances cireuses ou un mélange d'une ou plusieurs substances cireuses, caractérisé en ce que l'on épand sous forme de poudre les substances cireuses sur les granulés qui sont chauffés jusqu'à ramollissement de la substance cireuse.

2. Procédé selon la revendication 1, caractérisé en ce que l'on épand sous forme de poudre les substances cireuses mélangées à une ou plusieurs substances actives et éventuellement à un ou plusieurs adjuvants non-cireux.

3. Procédé selon la revendication 1, caractérisé en ce que, avant l'enrobage, on donne, par déformation plastique, aux granulés une forme sphérique par rotation prolongée et chauffage.

4. Procédé selon la revendication 1, caractérisé en ce que les granulés à enrober contiennent une substance cireuse hydrophobe ou un mélange de substances cireuses hydrophiles et hydrophobes.

5. Procédé selon la revendication 1, caractérisé en ce que l'on utilise comme substances cireuses des alcools gras ou leurs dérivés, des acides gras ou leurs dérivés, des hydrocarbures, des cires ou des polyéthylèneglycols et leurs dérivés de point de fusion supérieur à 20°C.